# EUROPEAN PATENT APPLICATION

(11) **EP 0 877 249 A1**
(43) Date of publication of application: **11.11.1998**
(21) Application number: 98303621.1
(22) Date of filing: 08.05.1998
(51) Int. Cl.: G01N 33/48

(54) **Star plots of analyte values over time**

(30) Priority: 09.05.1997 US 46063 P
(71) Applicant: Ortho-Clinical Diagnostics, Inc., Rochester, NY 14626-5101 (US)
(72) Inventor: Fleisher, Richard Barry, Pittsford, New York 14534 (US); Bullinger, Thomas Alan, Fairport, New York 14450 (US)
(74) Representative: Mercer, Christopher Paul

(57) **Abstract**

A method of displaying a historical record of patient analyte values as a single star plot or Kiviat graph. The method features plotting a different determined analyte value on each radii of the star plot, so that values of a particular date are connected by straight lines to form a polygon, and each polygon has a characteristic property of its boundary line that varies progressively with the age of the data. Representative characteristic properties include line density, line thickness and line color.

## Description

This invention relates to a method of plotting plural analyte values as they vary over time, as a star plot.

Kiviat, or star plots, are known for use in portraying multiple related parameters. A paper entitled "A Versatile Presentation of Parameters for Rapid Recognition of Total State", by J. Coekin, was submitted as part of the file history for US-A-4,675,147 published on June 23, 1987. That paper states that such star plots can be used as "diagrams peculiar to specific diseases", and that it has been suggested medical treatment "may be judged from regular measurements of several parameters such as temperature, blood pressure, heart rate, white count and so on" where the parameters "could be plotted along the axes of" a star plot.

Thus, it was recognized as early as 1987 that star plots readily lend themselves to a recognition of a disease state simply by reason of the star plot taking a particular shape that deviates from the healthy norm.

The importance of the change of the parameters over time is also noted in Coekin's paper. However, it proposes that such a change be "ascertained by comparing successive diagrams [star plots]". In other words, at times T₁, T₂, T₃ up to Tᵢ, values are ascertained and each plotted separately, and "i" number of plots are stacked together and flipped through to show changes over "i" dates.

Such a method of showing changes over time is awkward, and requires excessive pages and a visual comparison back and forth between a multitude of plots. There has been a need, therefore, to find an improved star plotting of patient variables such that time changes are more easily portrayed and compared.

We have devised a method of display that solves the aforementioned need.

More specifically, in accord with one aspect of the invention there is provided a method of displaying plural tested analyte values of a biological material, comprising the steps of:
a) determining in a clinical analyzer, the numerical value of each of a plurality of analytes of a single patient biological liquid;
b) plotting each of the determined values along a separate, unique radius emanating from a center point assigned a zero value, so that the plural radii create a star plot;
c) assigning and marking along each the radius, an indication of the normal values expected clinically for each of the plotted analytes; and
d) connecting with a straight line, the determined value of each analyte with that of the determined value of each adjacent analyte, creating a polygon, so that a comparison of the connecting lines against the normal values marked in step c) illustrates which determined analytes' values are abnormal;
characterized in that the method further includes the steps of e) repeating steps a) - d) for the same patient and the each analyte, at subsequent times to create a historical record of sets of polygons of sequentially determined values; and
f) plotting at least some of the polygons of sequentially determined values together in the same plot with a visual property of the straight lines of the polygons varying with age of the values.

In accord with another aspect of the invention, there is provided a method of diagnosing a patien= condition, comprising the steps of:
a) determining in a clinical analyzer, the value of analytes that are known to be indicative of the patient condition; and
b) plotting each of the determined values on a separate radius of a Kiviat graph, each of the radii having normal and abnormal ranges marked thereon and each determined analyte value being connected with a straight line to the determined analyte values adjacent thereto, creating a polygon;
characterized in that the method further includes the steps of c) repeating steps a) - b) at least once over time for the patient to create a historical record of polygons of sequentially determined values;
d) plotting the values for at least some of the polygons of sequential values onto a single graph depicting the polygons of sequential values instantaneously, with a visual property of the straight lines of the polygons varying with age of the values; and
e) comparing whether the shape created by the plots of step d) conform to a shape which, over time, 1) falls outside of acceptable values for the condition, or 2) is known to be indicative of a disease state.

Accordingly, it is an advantageous feature of the invention that the historical record of a patient's analytes can be ascertained from viewing a single star plot which contains the pertinent historical record in that single plot.

It is a related advantageous feature of the invention that the progression of a disease state over time, as shown by the shape of the star plot, is readily determinable by viewing a single star plot that has the pertinent historical record.

Other advantageous features will become apparent upon reference to the following Description when read in light of the attached drawings.

The file of this patent contains at least one drawing executed in color.
Fig. 1 is a representative star plot of seven blood analytes determined at a single point in time, of a hypothetical normal patient;
Fig. 2 is a representative star plot constructed in accordance with the invention, wherein both the line thickness and the line density vary progressively with age of the determined results;
Fig. 3 is a star plot similar to that of Fig. 2, but of a different embodiment of the invention wherein the color of the straight connecting lines varies progressively with the age of the determined results; and
Fig. 4 is a star plot similar to that of Fig. 1, but illustrating an example of how a shape of the plot alone is indicative of a particular disease state.

The invention is described in connection with certain preferred embodiments in which from five to seven examples of certain blood analyte values, determined on a preferred analyzer, are plotted in hard copy, showing a historical record wherein the age of each set of values is depicted by a change in either line thickness, line density, line color, or a combination of these. As used herein, "density" means the darkness of the line that is produced, which is achieved by reducing or increasing the pigment or ink content of the line. This in turn can be done by using dashed lines wherein the spacing between the dashes is increased or decreased, respectively, so that the most dense version is a solid line.

In addition, the invention is applicable regardless of: a) the biological material that is tested (for example, it could be tissue samples); b) how many different analytes, and which ones, are shown; c) the analyzer on which they are determined; d) whether the plot is shown on hard copy or a monitor screen produced electronically; and e) how the age is shown by the sequentially-varying characteristic properties of the connecting lines.

"Sequentially" as used herein does not mean necessarily that the data for the very next point in time is portrayed, one after the other. Rather, it means simply that the polygons represent sequential readings over time. Because of space constraints, it is expected that the user will select only those sequential intervals that are most appropriate for the particular patient and the particular suspected disease condition, skipping those data points that are not appropriate.

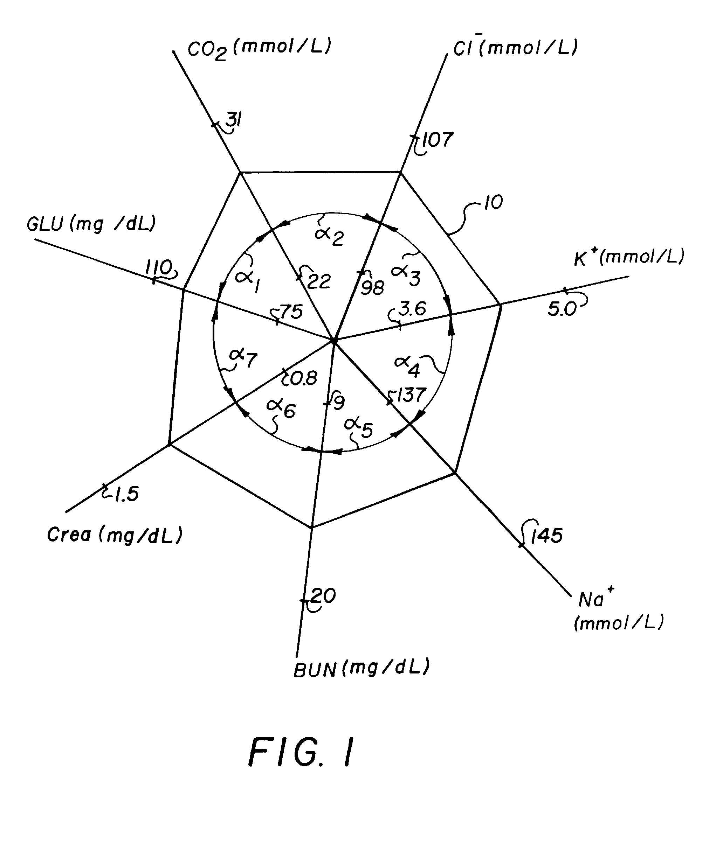

Fig. 1 is illustrative of the general process of using a star plot for the purposes of the invention. That is, starting from a center point assigned a zero value, a plurality of radii are extended therefrom equal in number to the selected analytes that are tested in a clinical analyzer for a particular patient, forming a star, each radius being angled from the adjacent radii by an angle alphaᵢ. As shown, seven different analytes were tested on a "VITROS 250"™ analyzer using dry slides, those analytes being, clockwise, Cl^{⊖}, K^{⊕}, Na^{⊕}, BUN, CREA, GLU, and CO₂, all conventional assays. The angle values for each alphaᵢ are arbitrary and can be the same or different, since they convey no clinical meaning.

Each radius is calibrated or marked somehow to depict the "normal" range, located approximately midway along the radius. The numerical values shown along each radius in Fig. 1 represent a generally accepted statement of the "normal" values for that analyte, more commonly called "reference intervals". As used herein, a "normal" value means, a value clinically expected from a patient who is considered healthy in the broadest context of disease management. "Disease" is used to include any ailment detrimental to the normal health of a patient, and includes physical trauma such as the presence of a kidney stone.

The range of "normal values" can be indicated in their actual numerical form, as shown in Fig. 1, or they can be normalized to some arbitrary value.

When the blood for the patient is tested, each of the values determined for the noted analyte is then plotted along the radius for that analyte. Those plotted determined values are then connected with a straight line, marked 10 in Fig. 1, and the resulting heptagon can be visually compared with the outer limits of the "normal" values to see which if any of the determined values is abnormal.

The result, however, depicts only the results of a single test on a single day in the life of the patient. To depict that patient's history of determined values, the method of the invention is as follows:

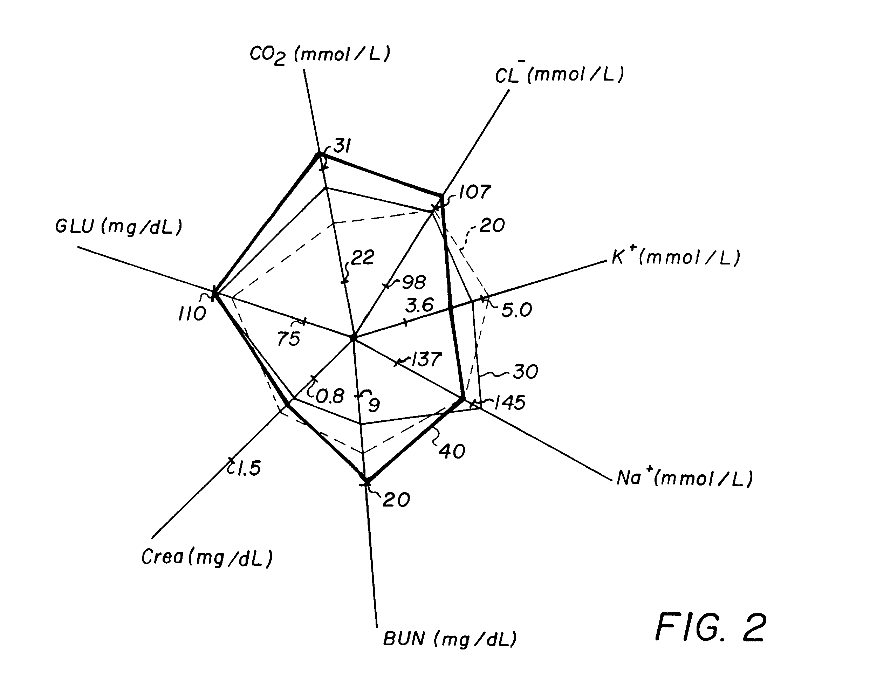

Turning to Fig. 2, the same star plot is shown as in Fig. 1, except that there are 3 sets of connecting lines, 20,30,40, each representing a different time in the life of the patient when the analyte values were determined. In accordance with the invention, one or more characteristic properties of lines 20,30,40 are varied progressively with the age of the determined values. In Fig. 2, two characteristic properties are selected to vary progressively, namely the line thickness and the line density. Thus, line 20 connects the values determined on the first test of the patient materials, and is the thinnest and least dense line. (Lesser density is produced by making the line in dashed form.) Line 30 connects the values determined after the first test, but before the last test, and has the properties of medium thickness and medium density. Line 40 connects the values determined after the test done to determine line 30, and hence connects the values that were determined most recently. It is the thickest and darkest line. Thus, by comparing the three sets of lines, a clinician or doctor can see the patient's trend, from the oldest to the newest values.

It will be readily appreciated that much more than three such heptagons produced by more than three connecting straight lines, with progressively varying properties, can be used to show more than three different times or dates of determined values. It will also be appreciated that the particular times or dates selected to be plotted will vary, depending on the need of the patient and the doctor, and can be readily selected as all, or as a subset of all, of the patient's determined values over time. However, a set less than three is probably not sufficient to show a trend, and a set greater than 10 is likely to be so obscuring in detail as to make the trend over time difficult to ascertain visually. Hence, the preferred number of connecting straight lines is from three to 10 for each star plot.

It will also be appreciated that a variety of conventional software programs can be used to produce the star plot of this invention, and to select which polygons are to be portrayed.

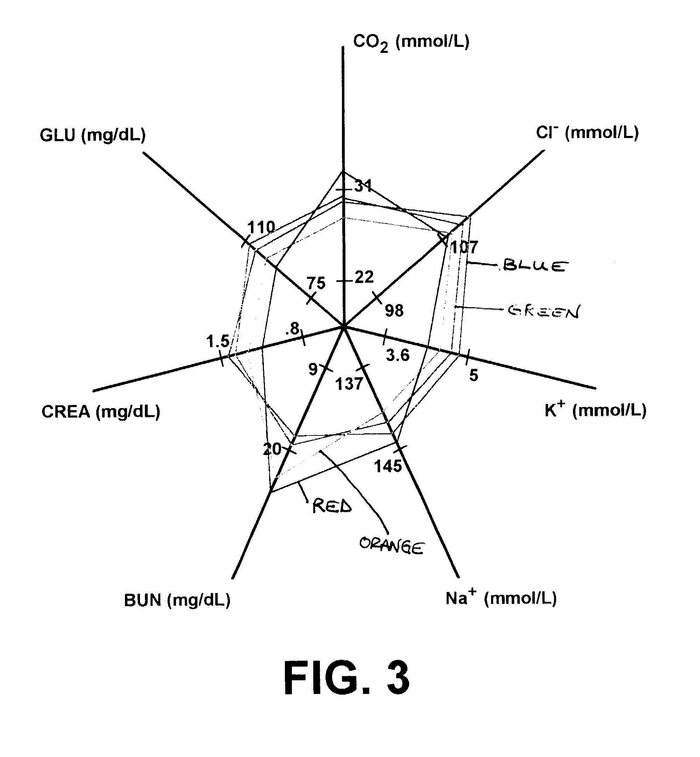

Alternatively, Fig. 3, the characteristic property of the connecting lines for each set of determined values can be color. As shown, line 20' for the oldest set of values is blue, line 30' for the second oldest is shown in green, line 40' for the second most recent (taken after the values of line 30') is shown in orange, and line 50 for the most recent set of determined values is shown in red. Hence, the age of the determined results is depicted by a color variance extending age-wise from the blue end (the oldest) of the color spectrum to the red end (the newest). The ends of course could be reversed in age, but here red was selected for the most recent determined values simply because it stands out and implies a warning. That is, red is thus a symbol that is most appropriate for the most recent patient results, given that "old" values are less connected to the patient's current health.

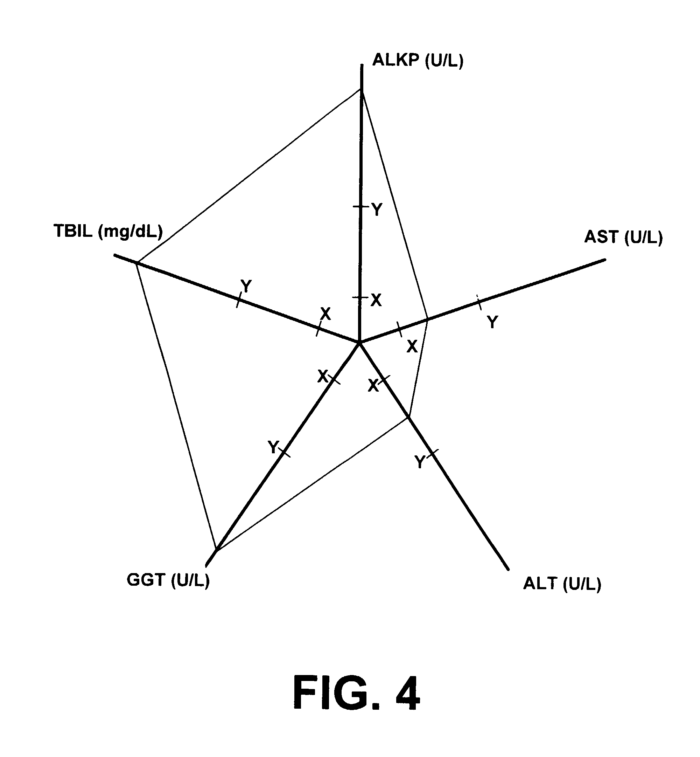

Fig. 4 is included by way of example as to how the shape of the star plot is indicative, in some cases, of a disease state. That is, it is known that the conventional analytes GGT, TBIL, AlkPhos, AST, and ALT are useful in measuring liver disease. ("GGT" stands for gamma glutamyltransferase, "TBIL" for total bilirubin, and "AlkPhos" for Alkaline Phosphatase.) If the first three of these are abnormally high, that is indicative of the disease condition of cholestasis. Fig. 4 shows such an abnormality, producing thus a characteristic shape of the polygon 10" that is easily recognized by the trained clinician or doctor, without examination of the minute details of the numerical values determined for each analyte. (The "normal" values are shown here as "x" and "y".) That is, the outward bulge along the axes for TBIL, AlkP, and GGT, with normal values only for AST and ALT, is indicative of the disease. What is not shown in Fig. 4, but is readily imagined, is a history of the same determined analytes, which, when produced by a method such as in Fig. 2 or Fig. 3, will show a more or less trend towards an improvement, or worsening, of the disease over time, again as indicated by the shape alone.

Other examples of disease state recognition in star plots can be found on pages 356 and 357 of the J. of Med. Tech., Volume 1, No. 5, May 1984.

## Claims

1. A method of displaying plural tested analyte values of a biological material, comprising the steps of:
a) determining in a clinical analyzer, the numerical value of each of a plurality of analytes of a single patient biological liquid;
b) plotting each of the determined values along a separate, unique radius emanating from a center point assigned a zero value, so that the plural radii create a star plot;
c) assigning and marking along each the radius, an indication of the normal values expected clinically for each of the plotted analytes; and
d) connecting with a straight line, the determined value of each analyte with that of the determined value of each adjacent analyte, creating a polygon, so that a comparison of the connecting lines against the normal values marked in step c) illustrates which determined analytes' values are abnormal;
characterized in that the method further includes the steps of e) repeating steps a) - d) for the same patient and the each analyte, at subsequent times to create a historical record of sets of polygons of sequentially determined values; and
f) plotting at least some of the polygons of sequentially determined values together in the same plot with a visual property of the straight lines of the polygons varying with age of the values.

2. A method of diagnosing a patient condition, comprising the steps of:
a) determining in a clinical analyzer, the value of analytes that are known to be indicative of the patient condition; and
b) plotting each of the determined values on a separate radius of a Kiviat graph, each of the radii having normal and abnormal ranges marked thereon and each determined analyte value being connected with a straight line to the determined analyte values adjacent thereto, creating a polygon;
characterized in that the method further includes the steps of c) repeating steps a) - b) at least once over time for the patient to create a historical record of polygons of sequentially determined values;
d) plotting the values for at least some of the polygons of sequential values onto a single graph depicting the some polygons of sequential values instantaneously, with a visual property of the straight lines of the polygons varying with age of the values; and
e) comparing whether the shape created by the plots of step d) conform to a shape which, over time, 1) falls outside of acceptable values for the condition, and 2) is known to be indicative of a disease state.

3. A method as defined in claim 1 or 2, wherein the varying visual property of the straight lines includes a progressive variance in thickness of the straight lines, over time.

4. A method as defined in claim 1 or 2, wherein the varying visual property of the straight lines includes a progressive variance in density of the straight lines, over time.

5. A method as defined in claim 1 or 2, wherein the varying visual property of the straight lines includes a progressive variance in color of the straight lines, over time.

6. A method as defined in claim 5, wherein the color variance proceeds from the blue end of the color spectrum for the oldest determined values, to the red end of the color spectrum for the most recent of the determined values.

7. A method as defined in claims 1 or 2, wherein the number of the some polygons formed by connected lines for each of the set of determined values is between 3 and 10.
